# EUROPÄISCHE PATENTANMELDUNG

(11) **EP 3 586 788 A1**
(43) Veröffentlichungstag der Anmeldung: **01.01.2020**
(21) Anmeldenummer: 19172731.2
(22) Anmeldetag: 06.05.2019
(51) Int. Cl.: A61B 90/70, A47L 15/50

(54) **KOPPLUNGSVORRICHTUNG ZUM MECHANISCHEN UND HYDRAULISCHEN KOPPELN EINES SPÜLGUTS MIT EINEM REINIGUNGSGERÄT, SPÜLGUTTRÄGER UND REINIGUNGSGERÄT MIT EINER KOPPLUNGSVORRICHTUNG**

(30) Priorität: 16.05.2018 DE 102018111693
(71) Anmelder: Miele & Cie. KG, 33332 Gütersloh (DE)
(72) Erfinder: Oebbeke, Dirk, 32547 Bad Oeynhausen (DE); Werner, Alexander, 33739 Bielefeld (DE)

(57) **Zusammenfassung**

Der hier vorgestellte Ansatz betrifft eine Kopplungsvorrichtung (105) zum mechanischen und hydraulischen Koppeln eines Spülguts mit einem Reinigungsgerät. Die Kopplungsvorrichtung (105) weist zumindest eine Spülgutverbindungseinrichtung (115), eine Geräteverbindungseinrichtung (120) und eine Riegeleinrichtung (200) auf. Die Spülgutverbindungseinrichtung (115) weist eine Schnittstelle zum Anschließen des Spülguts auf und ist mit der Geräteverbindungseinrichtung (120) koppelbar ausgeformt. Die Geräteverbindungseinrichtung (120) ist mit dem Reinigungsgerät verbindbar und mit der Spülgutverbindungseinrichtung (115) koppelbar ausgeformt. Die Riegeleinrichtung (200) ist dazu ausgeformt, um in einem Kopplungszustand (400) der Kopplungsvorrichtung (105), in dem die Spülgutverbindungseinrichtung (115) mit der Geräteverbindungseinrichtung (120) gekoppelt ist, die Spülgutverbindungseinrichtung (115) mit der Geräteverbindungseinrichtung (120) zu verriegeln.

## Beschreibung

Der hier vorgestellte Ansatz betrifft eine Kopplungsvorrichtung zum mechanischen und hydraulischen Koppeln eines Spülguts mit einem Reinigungsgerät, einen Spülgutträger mit einer Spülgutverbindungseinrichtung einer Kopplungsvorrichtung und ein Reinigungsgerät mit einer Kopplungsvorrichtung.

Für die Wiederverwendung von flexiblen Endoskopen sind für die Aufbereitung stets eine Reinigung und Desinfektion und gegebenenfalls noch eine Trocknung der inneren englumigen Kanäle notwendig. Um Spülflotte und/oder Trocknungsluft durch diese Kanäle leiten zu können, muss innerhalb des für die Aufbereitung vorgesehenen Reinigungs- und Desinfektionsgerätes, im folgenden RDG genannt, stets eine hydraulische Verbindung zwischen dem RDG und dem flexiblen Endoskop hergestellt werden. Dies kann vollautomatisch über entsprechende Aktorik und Sensorik erfolgen, aber auch manuell.

Die EP 2 201 886 B1 beschreibt einen Spülgutträger mit einem Anschlussstück für schlauchförmiges Reinigungsgut.

Dem hier vorgestellten Ansatz liegt die Aufgabe zugrunde, eine verbesserte Kopplungsvorrichtung zum mechanischen und hydraulischen Koppeln eines Spülguts mit einem Reinigungsgerät, einen Spülgutträger mit einer Spülgutverbindungseinrichtung einer verbesserten Kopplungsvorrichtung und ein Reinigungsgerät mit einer verbesserten Kopplungsvorrichtung zu schaffen.

Erfindungsgemäß wird diese Aufgabe durch eine Kopplungsvorrichtung zum mechanischen und hydraulischen Koppeln eines Spülguts mit einem Reinigungsgerät, ferner einen Spülgutträger mit einer Spülgutverbindungseinrichtung einer Kopplungsvorrichtung sowie ein Reinigungsgerät mit einer Kopplungsvorrichtung mit den Merkmalen der Hauptansprüche gelöst. Vorteilhafte Ausgestaltungen und Weiterbildungen des Ansatzes ergeben sich aus den nachfolgenden Unteransprüchen.

Die mit dem hier vorgestellten Ansatz erreichbaren Vorteile bestehen darin, dass durch eine hier vorgestellte Kopplungsvorrichtung eine manuelle, seitliche, sichere, dauerhafte, ergonomische und kostengünstige Ankopplung sowie Verriegelung eines Spülguts und/oder eines Spülgutträgers mit einem Reinigungsgerät realisierbar ist.

Eine Kopplungsvorrichtung zum mechanischen und hydraulischen Koppeln eines Spülguts mit einem Reinigungsgerät weist zumindest eine Spülgutverbindungseinrichtung, eine Geräteverbindungseinrichtung und eine Riegeleinrichtung auf. Die Spülgutverbindungseinrichtung weist eine Schnittstelle zum Anschließen des Spülguts auf und ist mit der Geräteverbindungseinrichtung koppelbar ausgeformt. Die Geräteverbindungseinrichtung ist mit dem Reinigungsgerät verbindbar und mit der Spülgutverbindungseinrichtung koppelbar ausgeformt. Die Riegeleinrichtung ist dazu ausgeformt, um in einem Kopplungszustand der Kopplungsvorrichtung, in dem die Spülgutverbindungseinrichtung mit der Geräteverbindungseinrichtung gekoppelt ist, die Spülgutverbindungseinrichtung mit der Geräteverbindungseinrichtung zu verriegeln.

Bei dem Reinigungsgerät kann es sich um ein Gerät zum Spülen und/oder Desinfizieren und/oder Trocknen von Spülgut handeln. Beispielsweise kann es sich bei dem Reinigungsgerät um ein sogenanntes "Reinigungs- und Desinfektionsgerät" handeln. Das Reinigungsgerät kann dazu ausgebildet sein, um schlauchförmiges Spülgut wie zumindest ein Endoskop zu reinigen, zu desinfizieren und/oder zu trocknen.

Die Riegeleinrichtung kann gemäß einer Ausführungsform mit der Geräteverbindungseinrichtung verbunden sein. So ist die Geräteverbindungseinrichtung zusammen mit der Riegeleinrichtung herstellbar.

Die Geräteverbindungseinrichtung kann eine Buchse zur Aufnahme zumindest einer Komponente der Spülgutverbindungseinrichtung in dem Kopplungszustand aufweisen. Dies ermöglicht eine kompakte und sichere Aufnahme der Spülgutverbindungseinrichtung, welche beispielsweise in die Geräteverbindungseinrichtung einsteckbar ausgeformt sein kann.

Die Riegeleinrichtung kann sich durch eine Öffnung der Geräteverbindungseinrichtung, beispielsweise eine Öffnung in der Buchse, erstrecken. Dabei können sich die Öffnung und/oder die Riegeleinrichtung quer zu einer Einführrichtung erstrecken, entlang der die Spülgutverbindungseinrichtung bei einem Überführen der Kopplungsvorrichtung in den Kopplungszustand in die Geräteverbindungseinrichtung eingeführt wird. Derart kann eine besonders stabile Verriegelung von einer Seite realisiert werden, die quer zu einer erwarteten Strömungsrichtung von Reinigungsfluid durch die Kopplungsvorrichtung greifen kann.

Von Vorteil ist es weiterhin, wenn die Riegeleinrichtung federbar lagerbar oder gelagert ist und die Spülgutverbindungseinrichtung eine schräge Seite, welche insbesondere von einer konisch zulaufenden Umfangsfläche gebildet sein kann und die dazu ausgeformt ist, um bei einem Überführen der Kopplungsvorrichtung in den Kopplungszustand, die Riegeleinrichtung automatisch vorzuspannen. Ein Koppeln und Verriegeln ist somit durch eine einzige manuell tätigbare Linearbewegung der Spülgutverbindungseinrichtung in die Geräteverbindungseinrichtung ermöglicht. Beim Verriegeln kann beispielsweise eine mit einer Feder gekoppelte Nase an der Riegeleinrichtung nach einem Überfahren über die schräge Seite bzw. die konisch zulaufende Umfangsfläche in eine der schrägen Seite bzw. der konisch zulaufenden Umfangsfläche nachgelagerte Ausnehmung an oder in der Spülgutverbindungseinrichtung einrasten.

Alternativ kann die Riegeleinrichtung aber auch mit der Spülgutverbindungseinrichtung verbunden sein. So ist die Spülgutverbindungseinrichtung zusammen mit der Riegeleinrichtung herstellbar.

Beispielsweise kann die Riegeleinrichtung eine Hebeleinrichtung aufweisen, die dazu ausgeformt ist, um durch eine manuelle Betätigung die Kopplungsvorrichtung in den Kopplungszustand zu überführen. So kann die Spülgutverbindungseinrichtung beispielsweise erst an die Geräteverbindungseinrichtung geführt werden und anschließend durch ein Betätigen der Hebeleinrichtung das Verriegeln bewirkt werden. Eine manuell betätigbare Hebeleinrichtung ist günstig in der Herstellung und verursacht keinerlei Kosten im Betrieb. Außerdem kann für einen Benutzer der Kopplungsvorrichtung so beim Verriegeln eine haptische Rückmeldung erzeugt werden, die den Benutzer vorteilhafterweise das sichere Verriegeln spüren lassen kann.

Die Hebeleinrichtung kann als eine Kniehebeleinrichtung oder ein Umlegehebel ausgeformt sein. Die Kniehebeleinrichtung kann durch ein drück- und/oder ziehbares Griffelement manuell betätigbar sein. Durch das Betätigen kann die Spülgutverbindungseinrichtung in oder an die Geräteverbindungseinrichtung gedrückt werden und dort in einem selbstgehemmten Zustand das Verriegeln bewirken. Der Umlegehebel kann nach einem Umlegen um beispielsweise 90° an der Geräteverbindungseinrichtung einrasten und so ein formschlüssiges Verriegeln bewirken.

Die Kopplungsvorrichtung kann eine Nase aufweisen, die dazu ausgeformt ist, um im Kopplungszustand in eine Ausnehmung der Kopplungsvorrichtung zu greifen, um die Kopplungsvorrichtung zu verriegeln. Wenn die Riegeleinrichtung mit der Geräteverbindungseinrichtung verbunden ist, kann die Nase wie oben bereits beschrieben, Teil der Riegeleinrichtung sein und in die Ausnehmung der Spülgutverbindungseinrichtung greifen. Wenn die Riegeleinrichtung als die Kniehebeleinrichtung an der Spülgutverbindungseinrichtung ausgeformt ist, kann die Nase an einem Ende eines Kniehebelgestänges der Kniehebeleinrichtung bei einem Betätigen des Griffelements in eine Freimachung der Kniehebeleinrichtung eingeführt werden und so eine Selbsthemmung bewirkt werden. Wenn die Riegeleinrichtung als der Umlegehebel ausgeformt ist, kann die Nase oder zumindest ein Bolzen an der Geräteverbindungseinrichtung angeordnet sein und zumindest eine Freimachung oder ein Haken an dem Umlegehebel beim Umlegen des Umlegehebels an den Bolzen greifen. Eine derartige Nase-Ausnehmung-Verbindung kann beim Betätigen der Riegeleinrichtung oder der Spülgutverbindungseinrichtung eine stabile mechanische und/oder formschlüssige Verbindung ermöglichen. Zudem kann eine derartige Verbindung beim Verriegeln eine haptische Rückmeldung erzeugen, die von einer Person spürbar ist. So kann der Person das Gefühl vermittelt werden, dass ein sicheres Verriegeln erfolgt ist.

Die Spülgutverbindungseinrichtung kann mit einem Spülgutträger verbindbar ausgeformt sein. Der Spülgutträger kann ein Spülkorb sein, der zur Aufnahme des Spülguts ausgeformt ist. Ein solcher Spülgutträger kann beispielsweise zur Aufnahme zumindest eines Endoskops oder zur Aufnahme einer Mehrzahl von Endoskopen ausgeformt sein. Durch ein Verbinden des Endoskops mit der Spülgutverbindungseinrichtung kann das Endoskop mechanisch und hydraulisch mit dem Reinigungsgerät verbunden werden.

Die Spülgutverbindungseinrichtung weist eine Fluidleitung und die Geräteverbindungseinrichtung eine weitere Fluidleitung auf, wobei die Fluidleitung im Kopplungszustand der Kopplungsvorrichtung mit der weiteren Fluidleitung fluidisch verbunden ist.

Von Vorteil ist es weiterhin, wenn die Spülgutverbindungseinrichtung mehrere, insbesondere zumindest drei Fluidleitungen und die Geräteverbindungseinrichtung mehrere, insbesondere zumindest drei weitere Fluidleitungen aufweist, wobei die Fluidleitungen im Kopplungszustand der Kopplungsvorrichtung mit den weiteren Fluidleitungen fluidisch verbunden sind. So können vorteilhafterweise mehrere, etwa drei oder mehr Endoskope unter Verwendung der Kopplungsvorrichtung gleichzeitig gereinigt, desinfiziert und/oder getrocknet werden.

Ein Spülgutträger weist eine Spülgutverbindungseinrichtung einer der vorgestellten Kopplungsvorrichtungen auf. Ein solcher Spülgutträger ist zur Verwendung mit einem Reinigungsgerät geeignet.

Ein Reinigungsgerät weist eine Kopplungsvorrichtung auf, die in einer der vorangehend vorgestellten Varianten ausgeformt ist, wobei die Geräteverbindungseinrichtung an einer Spülraumwand des Reinigungsgeräts angeordnet ist. Das Reinigungsgerät kann als ein Haushaltgerät oder ein gewerbliches oder professionelles Gerät ausgeformt sein, beispielsweise ein medizinisches Gerät wie ein Reinigungs- oder Desinfektionsgerät, ein Kleinsterilisator, ein Großraumdesinfektor oder eine Container-Waschanlage.

Ein hier vorgestelltes Reinigungsgerät kann als Ersatz für bekannte Reinigungsgeräte dienen, wobei das vorgestellte Reinigungsgerät vorteilhafterweise dank der Kopplungsvorrichtung deren Vorteile realisiert und somit ein schnelles und einfaches mechanisches und fluidisches Koppeln und Verriegeln eines Spülguts in dem Reinigungsgerät ermöglicht.

Ausführungsbeispiele des Ansatzes sind in den Zeichnungen rein schematisch dargestellt und werden nachfolgend näher beschrieben. Es zeigt
- Figur 1: eine schematische Querschnittdarstellung eines Reinigungsgeräts mit einer Kopplungsvorrichtung zum mechanischen und hydraulischen Koppeln eines Spülguts mit dem Reinigungsgerät gemäß einem Ausführungsbeispiel;
- Figur 2 bis 5: eine seitliche Querschnittdarstellung einer Kopplungsvorrichtung gemäß einem Ausführungsbeispiel;
- Figur 6 bis 9: eine seitliche Querschnittdarstellung einer Spülgutverbindungseinrichtung einer Kopplungsvorrichtung gemäß einem Ausführungsbeispiel;
- Figur 10: eine schematische Seitendarstellung einer Kopplungsvorrichtung gemäß einem Ausführungsbeispiel;
- Figur 11: eine schematische Vorderansicht einer Kopplungsvorrichtung gemäß einem Ausführungsbeispiel;
- Figur 12: eine schematische Aufsicht auf eine Kopplungsvorrichtung gemäß einem Ausführungsbeispiel;
- Figur 13: eine perspektivische Aufsicht auf einen Spülgutträger zur Verwendung mit einer Kopplungsvorrichtung gemäß einem Ausführungsbeispiel;
- Figur 14: eine perspektivische Vorderansicht einer Spülgutverbindungseinrichtung einer Kopplungsvorrichtung gemäß einem Ausführungsbeispiel;
- Figur 15: eine perspektivische Rückansicht einer Spülgutverbindungseinrichtung einer Kopplungsvorrichtung gemäß einem Ausführungsbeispiel;
- Figur 16: eine schematische Seitendarstellung einer Kopplungsvorrichtung gemäß einem Ausführungsbeispiel;
- Figur 17: eine schematische Vorderansicht einer Kopplungsvorrichtung gemäß einem Ausführungsbeispiel;
- Figur 18: eine schematische Aufsicht auf eine Kopplungsvorrichtung gemäß einem Ausführungsbeispiel;
- Figur 19: eine schematische Seitendarstellung einer Kopplungsvorrichtung gemäß einem Ausführungsbeispiel;
- Figur 20: eine schematische Vorderansicht einer Kopplungsvorrichtung gemäß einem Ausführungsbeispiel;
- Figur 21: eine schematische Aufsicht auf eine Kopplungsvorrichtung gemäß einem Ausführungsbeispiel; und
- Figur 22 bis 23: eine schematische Seitendarstellung einer Kopplungsvorrichtung gemäß einem Ausführungsbeispiel.

In der nachfolgenden Beschreibung günstiger Ausführungsbeispiele des vorliegenden Ansatzes werden für die in den verschiedenen Figuren dargestellten und ähnlich wirkenden Elemente gleiche oder ähnliche Bezugszeichen verwendet, wobei auf eine wiederholte Beschreibung dieser Elemente verzichtet wird.

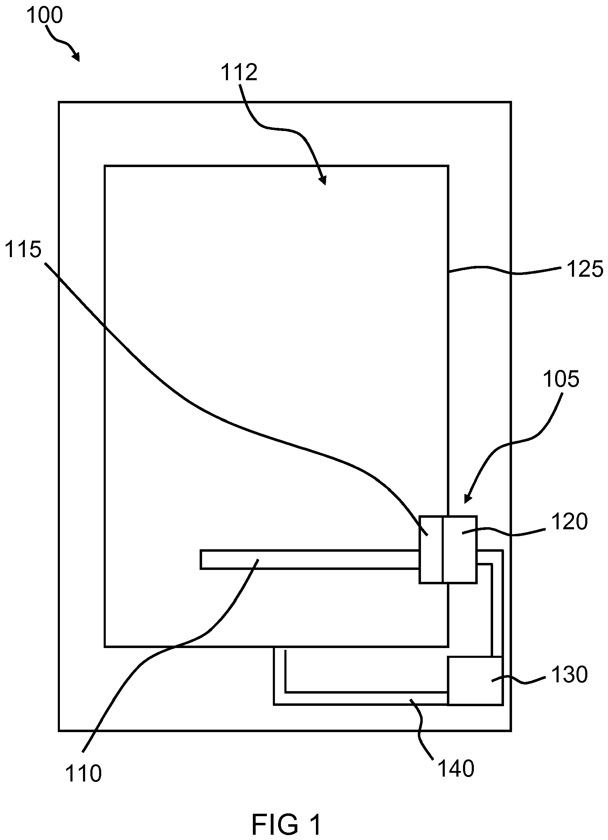

Figur 1 zeigt eine schematische Querschnittdarstellung eines Reinigungsgeräts 100 mit einer Kopplungsvorrichtung 105 zum mechanischen und hydraulischen Koppeln eines Spülguts 110 mit dem Reinigungsgerät 100 gemäß einem Ausführungsbeispiel.

Die Kopplungsvorrichtung 105 koppelt das in einem Spülraum 112 des Reinigungsgeräts 100 angeordnete Spülgut 110 mechanisch und hydraulisch mit dem Reinigungsgerät 100. Hierzu weist die Kopplungsvorrichtung 105 zumindest eine Spülgutverbindungseinrichtung 115, eine Geräteverbindungseinrichtung 120 und eine Riegeleinrichtung auf. Die Spülgutverbindungseinrichtung 115 weist eine Schnittstelle zum Anschließen des Spülguts 110 auf und ist gemäß diesem Ausführungsbeispiel mit der Geräteverbindungseinrichtung 120 gekoppelt. Die Geräteverbindungseinrichtung 120 ist gemäß diesem Ausführungsbeispiel mit dem Reinigungsgerät 100 verbunden und mit der Spülgutverbindungseinrichtung 115 gekoppelt. Die Riegeleinrichtung ist dazu ausgeformt, um in einem hier gezeigten Kopplungszustand der Kopplungsvorrichtung, in dem die Spülgutverbindungseinrichtung 115 mit der Geräteverbindungseinrichtung 120 gekoppelt ist, die Spülgutverbindungseinrichtung 115 mit der Geräteverbindungseinrichtung 120 zu verriegeln.

Die Geräteverbindungseinrichtung 120 der Kopplungsvorrichtung 105 ist an einer Spülraumwand 125 des Reinigungsgeräts 100 angeordnet. Bei der Spülraumwand 125 handelt es sich gemäß diesem Ausführungsbeispiel um eine Seitenwand des Spülraums 112 des Reinigungsgeräts 100. Bei dem Spülgut 110 handelt es sich gemäß diesem Ausführungsbeispiel um ein Endoskop. Das Reinigungsgerät 100 weist gemäß diesem Ausführungsbeispiel eine Reinigungsfluidbereitstellungseinrichtung 130 auf, die fluidisch mit der Geräteverbindungseinrichtung 120 der Kopplungsvorrichtung 105 verbunden ist, um ein Reinigen und/oder Desinfizieren und/oder Trocknen des Spülguts 110 zu ermöglichen. Außerdem weist die Reinigungsfluidbereitstellungseinrichtung 130 vorzugsweise eine fluidische Verbindung 140 zum Sammeltopf des Spülraumes 112 auf.

Die hier vorgestellte Kopplungsvorrichtung 105 kann auch als eine Vorrichtung zur Herstellung einer mechanischen und hydraulischen Verbindung zwischen einer Spülmaschine und einem Spülgutträger bezeichnet werden. Diese Verbindung ist vorteilhafterweise manuell herstellbar. Bei dem Reinigungsgerät 100 handelt es sich gemäß diesem Ausführungsbeispiel um ein sogenanntes "Reinigungs- und Desinfektionsgerät, kurz "RDG".

Es gibt Reinigungs- und Desinfektionsgeräte mit nur einer frontseitigen Tür zum Be- und Entladen des Gerätes sowie zweitürige RDGs, sogenannte Durchreichegeräte. Diese Geräte realisieren das Konzept "reine/unreine Seite", d. h., auf der einen Seite des RDGs wird über eine Tür das kontaminierte "unreine" Endoskop angeschlossen und in das Gerät geschoben und über die gegenüberliegende, zumeist auch noch baulich von der unreinen Seite getrennten Seite wird das aufbereitete "reine" Endoskop wieder entnommen. Dies reduziert das Risiko einer Rekontamination des Endoskopes. Bei diesem Typ von RDG erfolgt wie gemäß diesem Ausführungsbeispiel die Ankupplung in der Regel von der Seite, da keine Geräterückwand vorhanden ist.

Da die beschriebene hydraulische Ankupplung stets aufgrund des Spüldruckes und der Übertragungsquerschnitte in den hydraulischen Leitungen während des Aufbereitungsprozesses Kräften ausgesetzt ist, die zu einer Trennung der hydraulischen Ankupplung führen könnten, ist es eine Herausforderung, eine sichere und dauerhafte Haltung der hydraulischen Ankupplung herzustellen. Die in den nachfolgenden Figuren genauer beschriebenen Varianten der hier vorgestellten Kopplungsvorrichtung 105 realisieren oder ermöglichen jeweils eine manuelle, seitliche, sichere, dauerhafte, ergonomische und kostengünstige Ankupplung eines Spülgutträgers oder eines Spülguts 110 mit einem Reinigungsgerät 100.

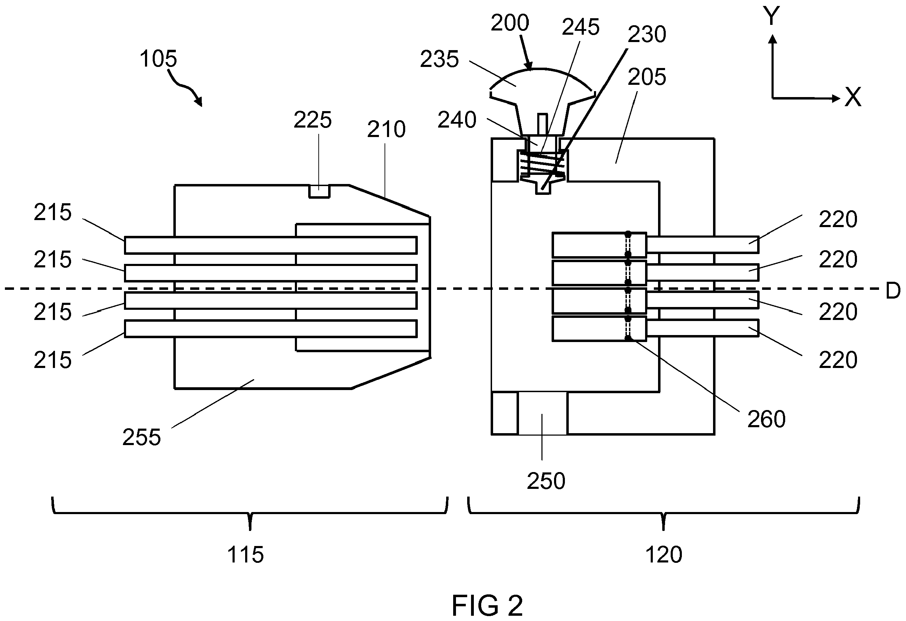

Figur 2 zeigt eine seitliche Querschnittdarstellung einer Kopplungsvorrichtung 105 gemäß einem Ausführungsbeispiel. Dabei kann es sich um die anhand von Figur 1 beschriebene Kopplungsvorrichtung 105 handeln, mit dem Unterschied, dass die Spülgutverbindungseinrichtung 115 und die Geräteverbindungseinrichtung 120 gemäß diesem Ausführungsbeispiel nicht in dem Kopplungszustand miteinander gekoppelt sind. Gemäß diesem Ausführungsbeispiel ist die Spülgutverbindungseinrichtung 115 zudem nicht mit dem Spülgut und die Geräteverbindungseinrichtung 120 nicht mit dem Reinigungsgerät verbunden.

Die Riegeleinrichtung 200 ist gemäß diesem Ausführungsbeispiel mit der Geräteverbindungseinrichtung 120 verbunden. Die Geräteverbindungseinrichtung 120 weist gemäß diesem Ausführungsbeispiel eine Buchse 205 zur Aufnahme zumindest einer Komponente der Spülgutverbindungseinrichtung 115 in dem Kopplungszustand auf. Die Riegeleinrichtung 200 erstreckt sich durch eine Öffnung der Buchse 205, wobei die Öffnung und die Riegeleinrichtung 200 sich quer zu einer Einführrichtung erstrecken, entlang der die Spülgutverbindungseinrichtung 115 bei einem Überführen der Kopplungsvorrichtung 105 in den Kopplungszustand in die Geräteverbindungseinrichtung 120 eingeführt wird.

Gemäß diesem Ausführungsbeispiel ist die Riegeleinrichtung 200 federbar gelagert und die Spülgutverbindungseinrichtung 115 weist eine schräge Seite 210, etwa eine konisch zulaufende Umfangsfläche, auf, die dazu ausgeformt ist, um bei einem Überführen der Kopplungsvorrichtung 105 in den Kopplungszustand, die Riegeleinrichtung 200 automatisch vorzuspannen.

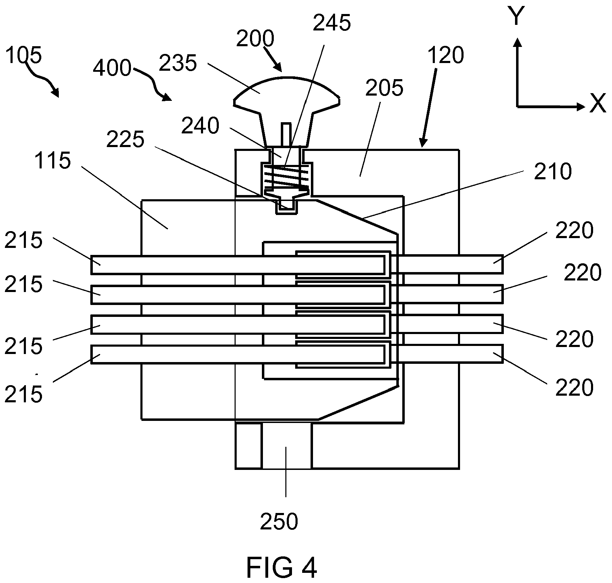

Optional weist die Spülgutverbindungseinrichtung 115 mehrere, etwa zumindest drei Fluidleitungen 215, gemäß diesem Ausführungsbeispiel vier der Fluidleitungen 215, und die Geräteverbindungseinrichtung 120 mehrere, etwa zumindest drei weitere Fluidleitungen 220, gemäß diesem Ausführungsbeispiel vier der weiteren Fluidleitungen 220, auf, wobei die Fluidleitungen 215 im Kopplungszustand der Kopplungsvorrichtung 105 mit den weiteren Fluidleitungen 220 fluidisch verbunden sind. Der Kopplungszustand ist in Figur 4 dargestellt.

Im Folgenden werden Ausführungsbeispiele anhand der Figur 1 nochmals mit anderen Worten beschrieben:
Für Endoskopreiniger, die eine seitliche Ankopplung zwischen dem Reinigungsgerät und dem Spülgutträger erfordern, realisiert die hier vorgestellte Kopplungsvorrichtung 105 eine innovative neue Lösung für diese Art von Systemen. Eine einheitliche Kupplung in mehreren Geräten zu integrieren, wird für eine ganzheitliche Endoskopaufbereitung immer wichtiger.

Vorteilhafterweise ist hierbei für das erfolgreiche Durchführen einer Ankopplung von mehreren, 1 bis n, Fluidwegen lediglich eine einzige Aktion notwendig. Zudem ist diese Aktion oder Aktivität sehr ergonomisch durchführbar, das heißt, es ist lediglich ein geringer Kraftaufwand beim Einstecken der Kupplung sowie eine sehr einfache manuell durchführbare Aktion zum Verriegeln vonnöten, nämlich lediglich ein Einstecken der Spülgutverbindungseinrichtung 115 in die Geräteverbindungseinrichtung 120.

Die hier und nachfolgend aufgezeigten Varianten einer Kopplungsvorrichtung 105 gestalten einen Kupplungsvorgang wesentlich benutzerfreundlicher und ergonomischer, als dies bei manuellen Ankupplungen bisher der Fall gewesen ist. Dies wird unter Verwendung der hier vorgestellten Kopplungsvorrichtung 105 vorteilhafterweise durch einen geringen Kraftaufwand sowie eine minimale Anzahl an Benutzeraktionen, <=1, erzielt.

Eine in den Figuren 2 bis 5 gezeigte "Lösung 1" der Kopplungsvorrichtung 105 zeigt ein System bestehend aus einer Spülgutverbindungseinrichtung 115 in Form eines Steckers und einer Geräteverbindungseinrichtung 120 in Form einer fest verbauten Buchse, welche eine Anzahl von 1 bis n Kanälen oder Fluidleitungen 215, 220, durch die ein Fluid geleitet werden kann, verbinden und trennen kann. Im hier skizzierten Ausführungsbeispiel sind die vier Kanäle des Steckers von den vier Kanälen der Buchse getrennt, in Figur 4 sind sie verbunden.

Der Stecker weist gemäß diesem Ausführungsbeispiel eine Ausnehmung 225 oder Ausformung auf, welche für die Funktion des Verriegelns erforderlich ist. Zudem besitzt der Stecker die schräge Seite 210, bzw. konisch zulaufende Umfangsfläche, auch "Abschrägung" genannt, welche für die gemäß diesem Ausführungsbeispiel automatische Verriegelung zwingend erforderlich ist.

An der Buchse 205 der Kopplungsvorrichtung 105 gibt es die Riegeleinrichtung 200 zum Verriegeln des Steckers, wobei gemäß diesem Ausführungsbeispiel eine Nase 230 an der Riegeleinrichtung 200 dazu ausgeformt ist, um in die Ausnehmung 225 am Stecker einzugreifen. Die Riegeleinrichtung 200, die auch als "Verriegelung" bezeichnet werden kann, ist gemäß diesem Ausführungsbeispiel Bestandteil der Geräteverbindungseinrichtung 120. Die Riegeleinrichtung 200 weist gemäß diesem Ausführungsbeispiel ein Griffteil 235 auf, das auf einen Stößel 240 der Riegeleinrichtung 200 geschraubt ist. Beim Einbau wird zudem eine Feder 245 um einen Schaft des Stößels 240 gelegt, dann der Stößel 240 mit der Feder 240 durch einen Grundkörper der Buchse 205 gesteckt und mit dem Griffteil 235 verschraubt. Um eine Stufenbohrung, in der die Riegeleinrichtung 200 gemäß diesem Ausführungsbeispiel geführt ist, fertigen zu können, gibt es an einer Stelle der Buchse 205 eine Querbohrung 250, welche die Fertigung eines Absatzes in der Buchse 205 im Bereich der Riegeleinrichtung 200 ermöglicht hat. Positiver Nebeneffekt dieser Querbohrung 250 ist, dass bei einem Abkoppeln ausströmendes Fluid wie Wasser, Spülflotten und/oder Chemikalien durch selbige abfließen kann.

Um beide Hälften, also den Stecker und die Buchse der Kopplungsvorrichtung 105 und somit auch die Fluidleitungen 215, 220 miteinander zu verbinden, muss ein Anwender den Stecker parallel zur Achse D in Richtung X auf die Buchse 205 bewegen. Bevor die Fluidleitungen 215, 220 vollständig verbunden sind, gleitet während dieses Ankoppelvorgangs die Riegeleinrichtung 200, die auch als Verriegelungseinrichtung bezeichnet werden kann, speziell die Nase 230 am Stößel 240, auf der Oberfläche der schrägen Seite 210 eines Grundkörpers 255 des Steckers. Während dies geschieht, wird die Feder 245 gestaucht und die Riegeleinrichtung 200 in Y-Richtung verschoben. Sobald der Stecker vollständig angekoppelt ist, drückt die Feder 245 die Riegeleinrichtung 200 entlang der Y-Richtung in die Ausnehmung 225 des Grundkörpers 255 des Steckers. Der nun vorliegende Formschluss, in Figur 4 gezeigt, führt dazu, dass ein unbeabsichtigtes Lösen der Kupplungsteile im Kopplungszustand nicht möglich ist. Auch bei anliegendem Überdruck in den Fluidleitungen 215, 220, entsprechend des üblichen Betriebsdrucks, führt dies zu keiner Trennung der Kupplung zwischen der Spülgutverbindungseinrichtung 115 und der Geräteverbindungseinrichtung 120.

Eine Abdichtung der beiden Teile eines Fluidkanals aus Fluidleitung 215 und weiterer Fluidleitung 220 erfolgt gemäß diesem Ausführungsbeispiel jeweils über ein Dichtelement 260, das kurz vor Erreichen der Endlage "angekoppelt" seine vollständige Wirkung zeigt. Das Dichtelement 260 ist gemäß einem alternativen Ausführungsbeispiel im Teil der Fluidleitung 215 im Stecker, also innerhalb der Fluidleitungen 215 oder, wie gemäß diesem Ausführungsbeispiel, in der Buchse 205, also innerhalb der weiteren Fluidleitung 220, integriert.

In Bezug auf Lösung 1 ergibt sich der Vorteil, dass beim Ankoppeln des Steckers an die geräteseitige Buchse 205 die Verriegelung automatisch durch das Aufstecken beider Bauteile erfolgt. Eine zusätzliche Benutzeraktion ist für das Verriegeln beim Verbinden der Kupplungshälften nicht mehr erforderlich. Die Art der Verriegelung wird durch einen Formschluss realisiert, ein versehentliches Lösen der Kupplung ist somit ausgeschlossen. Die Steckkräfte beim Verbinden beider Kupplungshälften sind auf ein Minimum reduziert, da nur kurz vor Erreichen der Endlage "angekoppelt" die durch die verbauten Dichtelemente 260 auftretenden Kräfte überwunden werden müssen. Durch das automatische Verriegeln ist für den Anwender zudem sofort und intuitiv erkennbar, ob der Ankupplungsvorgang erfolgreich verlaufen ist. Zudem ist eine solche manuelle Ankupplung wesentlich kostengünstiger als eine mögliche automatisch ausgeführte Ankupplung. Dies betrifft sowohl die hardwaretechnischen als auch die steuerungstechnischen Kostenaufwände.

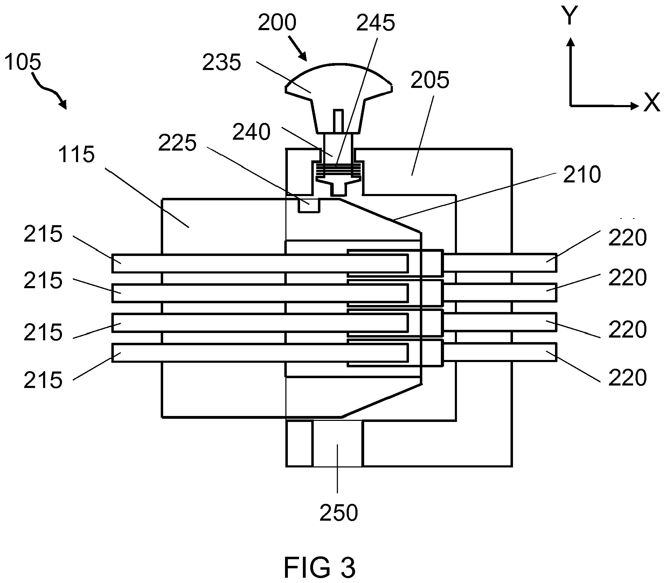

Figur 3 zeigt eine seitliche Querschnittdarstellung einer Kopplungsvorrichtung 105 gemäß einem Ausführungsbeispiel. Dabei handelt es sich um die in Figur 2 beschriebene Kopplungsvorrichtung 105, mit dem Unterschied, dass die Spülgutverbindungseinrichtung 115 während eines Ankoppelvorgangs teilweise in die Buchse 205 eingeführt dargestellt ist. Nach einem Überfahren über die schräge Seite 210 ist die Riegeleinrichtung 200 gemäß diesem Ausführungsbeispiel in einem nach oben gedrückten Zustand dargestellt, in dem die Feder 245 gespannt ist.

Figur 4 zeigt eine seitliche Querschnittdarstellung einer Kopplungsvorrichtung 105 gemäß einem Ausführungsbeispiel. Dabei handelt es sich um die in Figur 3 beschriebene Kopplungsvorrichtung 105, mit dem Unterschied, dass die Kopplungsvorrichtung 105 nun im Kopplungszustand 400 angeordnet ist.

Gemäß diesem Ausführungsbeispiel greift hierbei im Kopplungszustand 400 der Kopplungsvorrichtung 105 die Nase der Riegeleinrichtung 200 in die Ausnehmung 225 der Spülgutverbindungseinrichtung 115, um die Kopplungsvorrichtung 105 zu verriegeln.

Soll die Kupplung wieder getrennt werden, das heißt, der Kopplungszustand 400 der Kopplungsvorrichtung 105 gelöst werden, so kann der Anwender die Riegeleinrichtung 200 in Y-Richtung ziehen und den Stecker entgegen der X-Richtung aus der Buchse 205 führen. Sobald die Endlage im angekoppelten Zustand verlassen wird, kann die Riegeleinrichtung 200 losgelassen werden.

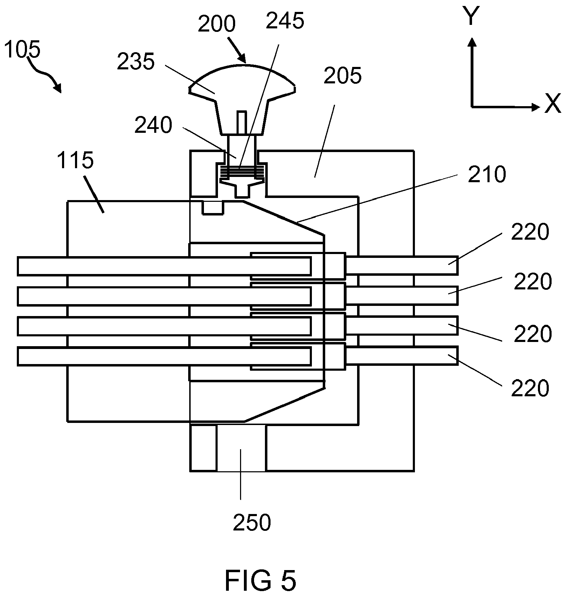

Figur 5 zeigt eine seitliche Querschnittdarstellung einer Kopplungsvorrichtung 105 gemäß einem Ausführungsbeispiel. Dabei handelt es sich um die in Figur 4 beschriebene Kopplungsvorrichtung 105, mit dem Unterschied, dass die Kopplungsvorrichtung 105 entsprechend der in Figur 4 beschriebenen Weise wieder aus dem Kopplungszustand gelöst wurde.

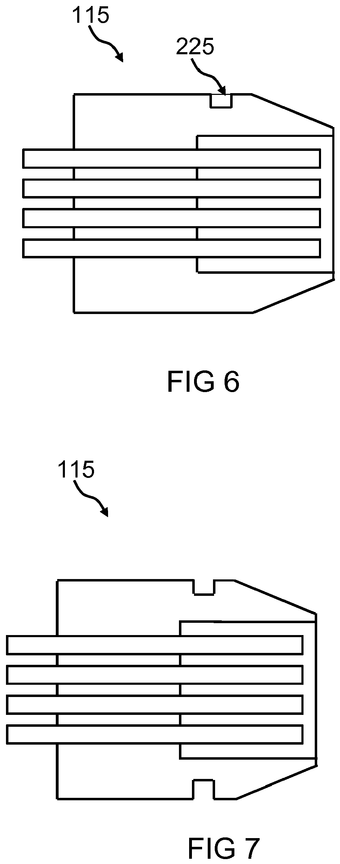

Figur 6 zeigt eine seitliche Querschnittdarstellung einer Spülgutverbindungseinrichtung 115 einer Kopplungsvorrichtung gemäß einem Ausführungsbeispiel. Dabei handelt es sich um die in einer der Figuren 2 bis 5 beschriebene Spülgutverbindungseinrichtung 115 in Form eines Steckers. Die Ausnehmung 225 oder Ausformung der Spülgutverbindungseinrichtung 115 ist gemäß diesem Ausführungsbeispiel durch eine Frästasche realisiert.

Figur 7 zeigt eine seitliche Querschnittdarstellung einer Spülgutverbindungseinrichtung 115 einer Kopplungsvorrichtung gemäß einem Ausführungsbeispiel. Dabei handelt es sich um die in Figur 6 beschriebene Spülgutverbindungseinrichtung 115, mit dem Unterschied, dass die Ausnehmung oder Ausformung gemäß diesem Ausführungsbeispiel durch einen angedrehten Einstich realisiert ist.

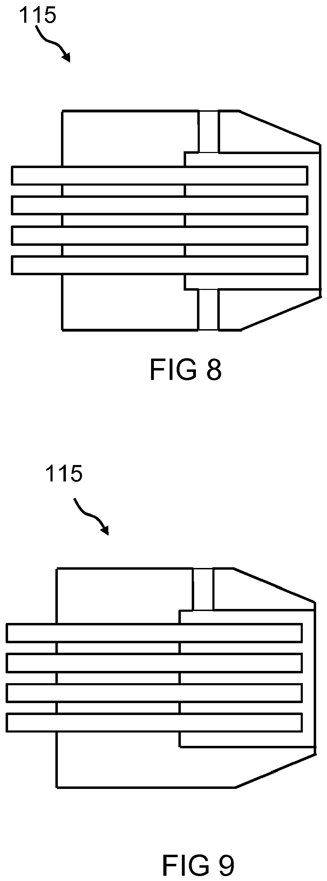

Figur 8 zeigt eine seitliche Querschnittdarstellung einer Spülgutverbindungseinrichtung 115 einer Kopplungsvorrichtung gemäß einem Ausführungsbeispiel. Dabei handelt es sich um die in Figur 7 beschriebene Spülgutverbindungseinrichtung 115, mit dem Unterschied, dass die Ausnehmung oder Ausformung gemäß diesem Ausführungsbeispiel durch eine Querbohrung realisiert ist.

Figur 9 zeigt eine seitliche Querschnittdarstellung einer Spülgutverbindungseinrichtung 115 einer Kopplungsvorrichtung gemäß einem Ausführungsbeispiel. Dabei handelt es sich um die in Figur 8 beschriebene Spülgutverbindungseinrichtung 115, mit dem Unterschied, dass die Ausnehmung oder Ausformung gemäß diesem Ausführungsbeispiel durch eine Querbohrung, die als Sackloch-Bohrung ausgestaltet ist, realisiert ist.

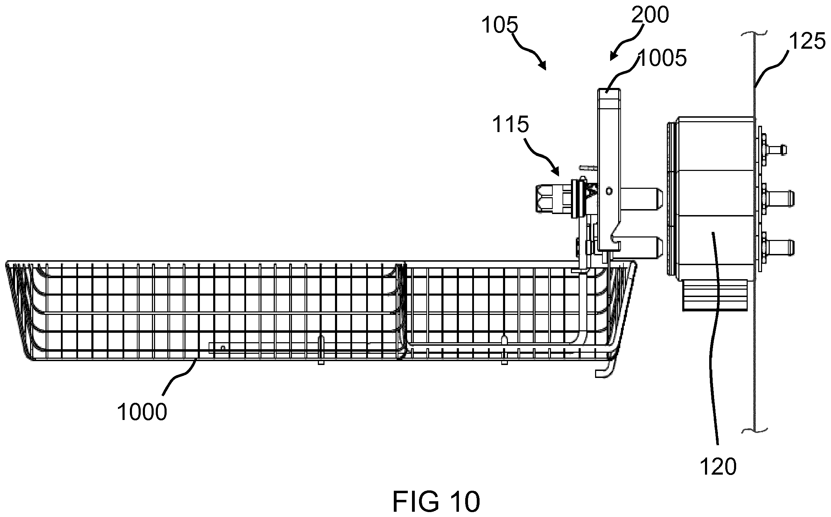

Figur 10 zeigt eine schematische Seitendarstellung einer Kopplungsvorrichtung 105 gemäß einem Ausführungsbeispiel. Dabei kann es sich um die in Figur 1 beschriebene Kopplungsvorrichtung 105 handeln, mit dem Unterschied, dass die Spülgutverbindungseinrichtung 115 und die Geräteverbindungseinrichtung 120 gemäß diesem Ausführungsbeispiel nicht in dem Kopplungszustand miteinander gekoppelt sind. Gemäß diesem Ausführungsbeispiel ist die Spülgutverbindungseinrichtung 115 zudem nicht mit dem Spülgut, sondern mit einem Spülgutträger 1000 in Form eines Spülkorbs verbunden.

Gemäß diesem Ausführungsbeispiel ist die Riegeleinrichtung 200 mit der Spülgutverbindungseinrichtung 115 verbunden. Die Riegeleinrichtung 200 weist gemäß diesem Ausführungsbeispiel eine Hebeleinrichtung auf, die dazu ausgeformt ist, um durch eine manuelle Betätigung die Kopplungsvorrichtung 105 in den Kopplungszustand zu überführen. Die Hebeleinrichtung ist gemäß diesem Ausführungsbeispiel als ein Umlegehebel 1005 ausgeformt.

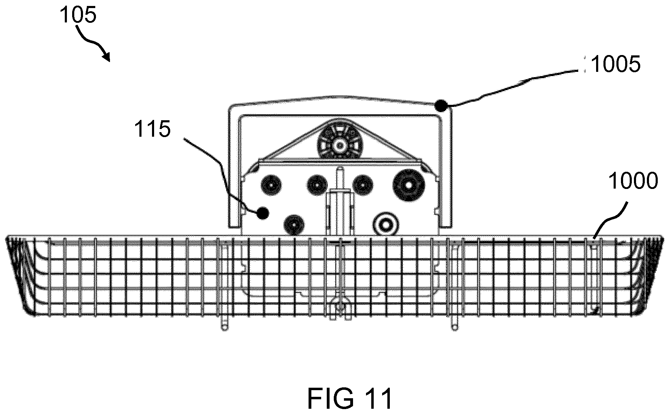

Figur 11 zeigt eine schematische Vorderansicht einer Kopplungsvorrichtung 105 gemäß einem Ausführungsbeispiel. Dabei handelt es sich um die in Figur 10 beschriebene Kopplungsvorrichtung 105.

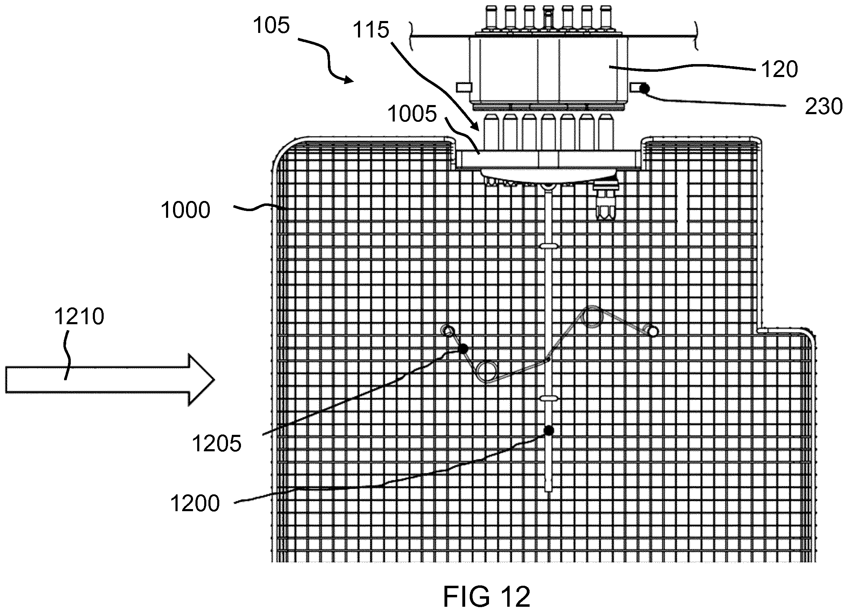

Figur 12 zeigt eine schematische Aufsicht auf eine Kopplungsvorrichtung 105 gemäß einem Ausführungsbeispiel. Dabei handelt es sich um die in der Figur 11 oder 12 beschriebene Kopplungsvorrichtung 105.

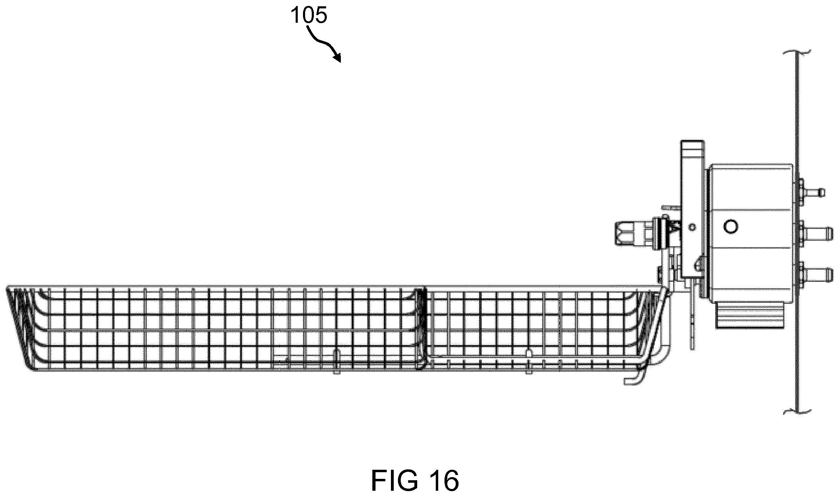

Eine in den Figuren 10 bis 21 gezeigte "Lösung 2" der Kopplungsvorrichtung 105 zeigt ein System bestehend aus dem Spülgutträger 1000 und/oder der darin gelagerten oder lagerbaren und geführten oder führbaren Spülgutverbindungseinrichtung 115 in Form eines Anschlussstückes. Eine Lagerung der Spülgutverbindungseinrichtung 115 ist gemäß diesem Ausführungsbeispiel über einen Führungsdraht 1200 und über zwei Schenkelfedern 12005 bistabil ausgeführt, das heißt, es gibt zwei stabile Endlagen. Anders als derartige bekannte Systeme weist die Spülgutverbindungseinrichtung 115 die Hebeleinrichtung mit dem Umlegehebel 1005 auf, der auch gleichzeitig von dem Anwender als Griff nutzbar ist. Die Figuren 10 bis 12 zeigen einen eingeschobenen Zustand des Spülgutträgers 1000 in das Reinigungsgerät. In dieser Position befindet sich die Spülgutverbindungseinrichtung 115 genau gegenüber der geräteseitigen und in der Spülraumwand angeordneten Geräteverbindungseinrichtung 120. In dieser Position kann nun der Anwender manuell die Spülgutverbindungseinrichtung 115 in die Geräteverbindungseinrichtung 120 bewegen, wodurch die hydraulische Verbindung des Spülgutverbindungseinrichtung 115 zur Geräteverbindungseinrichtung 120 hergestellt ist, siehe Figuren 16 bis 18.

An zwei gegenüberliegend angeordneten Seiten weist die Geräteverbindungseinrichtung 120 gemäß diesem Ausführungsbeispiel je eine Nase 230 in Form eines Bolzens auf. Ein Pfeil 1210 zeigt eine Einschubrichtung des Spülgutträgers 1000 und/oder der Spülgutverbindungseinrichtung 115.

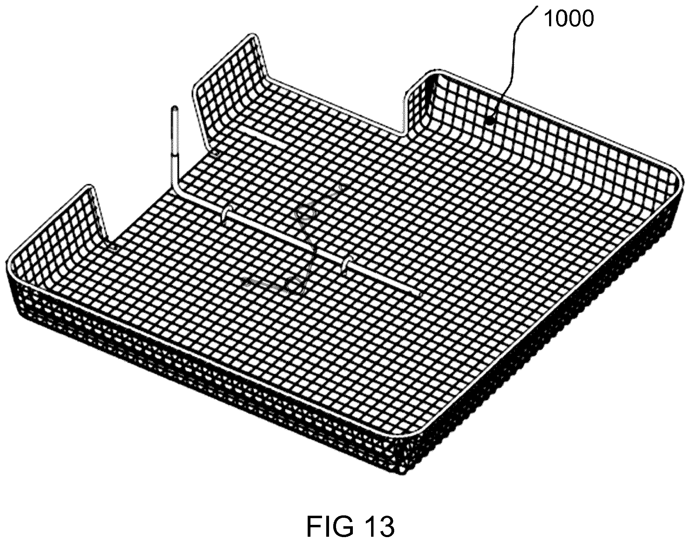

Figur 13 zeigt eine perspektivische Aufsicht auf einen Spülgutträger 1000 zur Verwendung mit einer Kopplungsvorrichtung gemäß einem Ausführungsbeispiel. Dabei kann es sich um einen der in den Figuren 10 bis 12 beschriebenen Spülgutträger 1000 handeln.

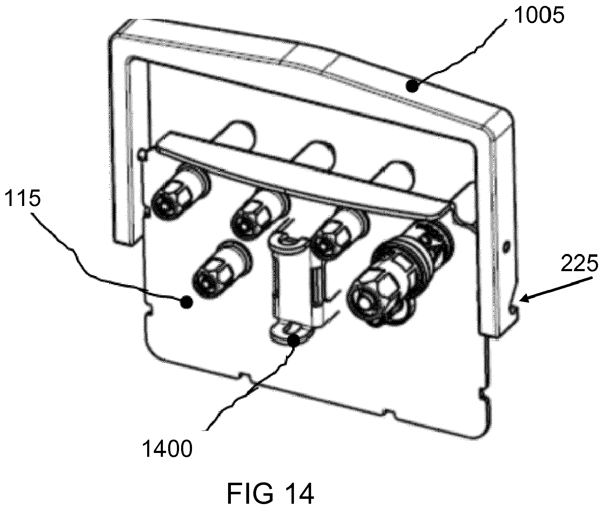

Figur 14 zeigt eine perspektivische Vorderansicht einer Spülgutverbindungseinrichtung 115 einer Kopplungsvorrichtung gemäß einem Ausführungsbeispiel. Dabei kann es sich um die in den Figuren 10 bis 12 beschriebene Spülgutverbindungseinrichtung 115 handeln.

Eine Verbindungseinrichtung 1400 ist zum Verbinden der Spülgutverbindungseinrichtung 115 mit dem in den Figuren 12 und 13 gezeigten Führungsdraht ausgeformt. An zwei gegenüberliegend angeordneten Seiten weist der Umlegehebel 1005 gemäß diesem Ausführungsbeispiel je eine Ausnehmung 225 in Form einer Freimachung auf.

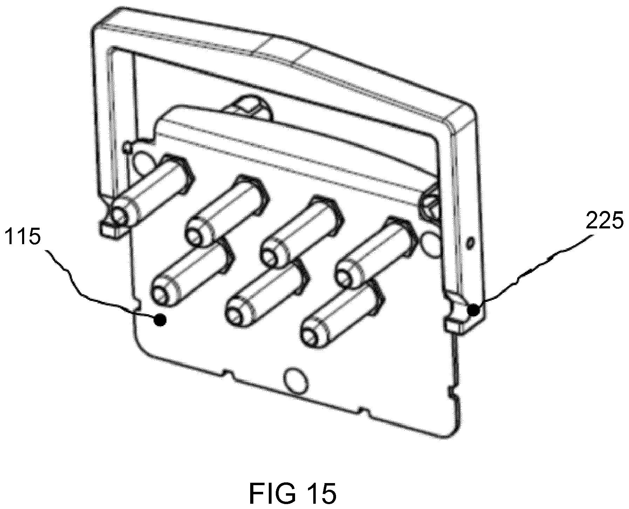

Figur 15 zeigt eine perspektivische Rückansicht einer Spülgutverbindungseinrichtung 115 einer Kopplungsvorrichtung gemäß einem Ausführungsbeispiel. Dabei handelt es sich um die in Figur 14 beschriebene Spülgutverbindungseinrichtung 115.

Figur 16 zeigt eine schematische Seitendarstellung einer Kopplungsvorrichtung 105 gemäß einem Ausführungsbeispiel. Dabei kann es sich um die in Figur 10 gezeigte Kopplungsvorrichtung 105 mit der in Figur 12 beschriebenen Geräteverbindungseinrichtung und der in den Figuren 14 und 15 beschriebenen Spülgutverbindungseinrichtung handeln, mit dem Unterschied, dass die Spülgutverbindungseinrichtung manuell in die Geräteverbindungseinrichtung bewegt wurde, wodurch die hydraulische Verbindung der Spülgutverbindungseinrichtung zur Geräteverbindungseinrichtung hergestellt wurde.

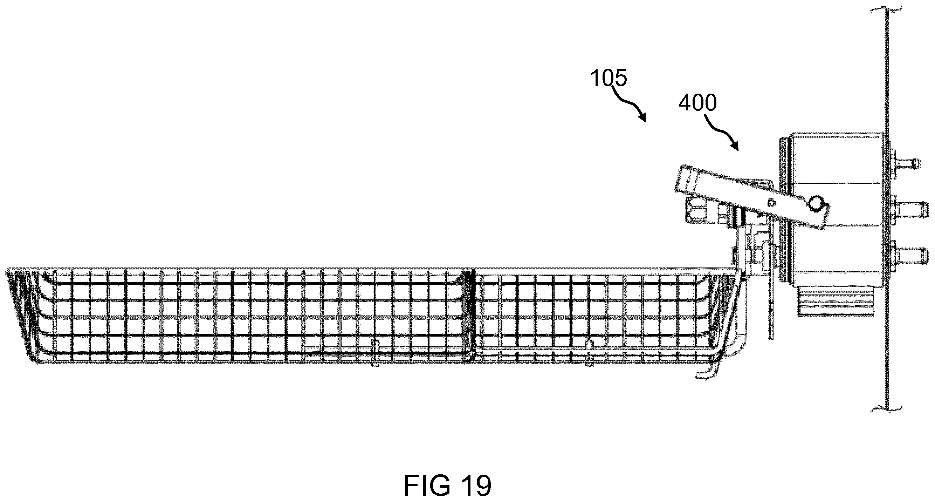

Zur Realisierung einer mechanisch sicheren und dauerhaften Ankupplung kann nun der Umlegehebel durch den Anwender in der Art umgelegt werden, dass die Freimachungen des Umlegehebels in der Art in die Bolzen der Geräteverbindungseinrichtung greifen, dass das Anschlussstück, also die Spülgutverbindungseinrichtung, sicher arretiert ist, siehe hierzu die Figuren 19 bis 21.

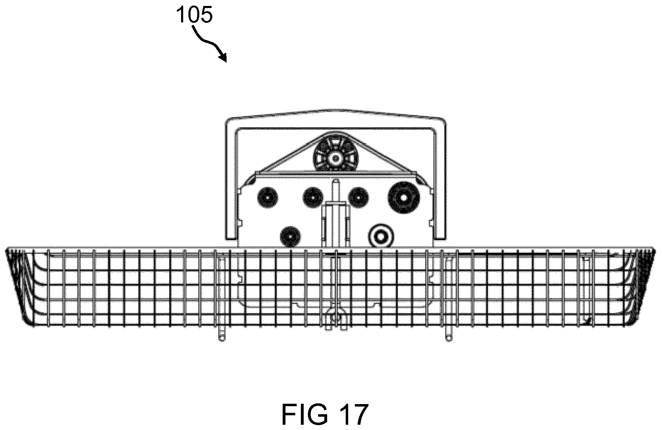

Figur 17 zeigt eine schematische Vorderansicht einer Kopplungsvorrichtung 105 gemäß einem Ausführungsbeispiel. Dabei handelt es sich um die in Figur 16 beschriebene Kopplungsvorrichtung 105.

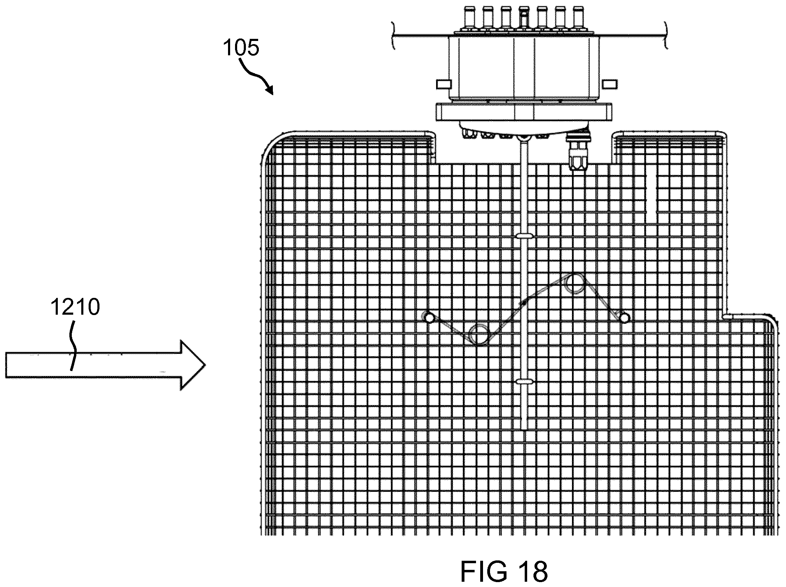

Figur 18 zeigt eine schematische Aufsicht auf eine Kopplungsvorrichtung 105 gemäß einem Ausführungsbeispiel. Dabei handelt es sich um die in der Figur 16 oder 17 beschriebene Kopplungsvorrichtung 105.

Figur 19 zeigt eine schematische Seitendarstellung einer Kopplungsvorrichtung 105 gemäß einem Ausführungsbeispiel. Dabei handelt es sich um die in der Figur 16 bis 18 beschriebene Kopplungsvorrichtung 105, mit dem Unterschied, dass die Kopplungsvorrichtung 105 in dem Kopplungszustand 400 angeordnet ist.

Hierzu ist der Umlegehebel manuell umgelegt worden, wodurch die Ausnehmungen des Umlegehebels an die Nasen der Geräteverbindungseinrichtung greifen, um die Kopplungsvorrichtung 105 zu verriegeln.

Im Kopplungszustand 400 des Anschlussstückes und des Umlegehebels kann nach einem Schließen einer Gerätetür des Reinigungsgeräts ein Aufbereitungsprozess gestartet werden. Zur Entnahme des Spülgutträgers erfolgt das Lösen des Anschlussstückes in umgekehrter Reihenfolge.

Im Vergleich zu einer möglichen motorischen Ankupplungsvorrichtung ergeben sich bei der hier vorgestellten Kopplungsvorrichtung 105 als Hauptvorteile geringere Herstellkosten und es fallen geringere Entwicklungskosten an, da kein Aktor, keine Sensorik, kein Aufwand in der Steuerung sowie ein geringerer Aufwand in der Approbation nötig ist, da keine motorische Automatik benötigt wird, Stichwort "Einklemmschutz", "Arbeitssicherheit", "Mechanische Gefährdung".

Weiter ist es möglich, ein bereits bestehendes und bewährtes Serienkonzept, bestehend aus einem Anschlussstück, zu übernehmen, d. h., der Anwender muss sich in Bezug auf die Verwendung des Anschlussstückes nicht umgewöhnen.

Weiter sind wie bei "Lösung 1" die Kupplungskräfte auf ein Minimum reduziert, da über den Umlegehebel aufgrund des relativ langen Hebelarms große Kräfte aufgebracht werden können und somit ein vergleichsweise geringer Kraftaufwand durch den Anwender nötig ist. Zusätzlich erhält der Anwender durch das Verrasten des Umlegehebels mit den Bolzen der Geräteverbindungseinrichtung eine haptische Rückmeldung über den sicheren und erfolgreichen Kupplungsvorgang.

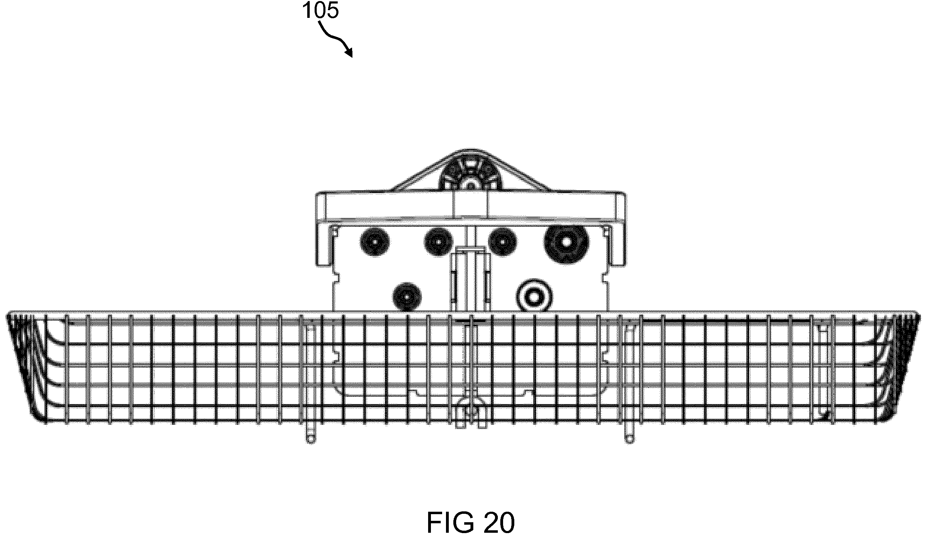

Figur 20 zeigt eine schematische Vorderansicht einer Kopplungsvorrichtung 105 gemäß einem Ausführungsbeispiel. Dabei handelt es sich um die in Figur 19 beschriebene Kopplungsvorrichtung 105 in dem Kopplungszustand.

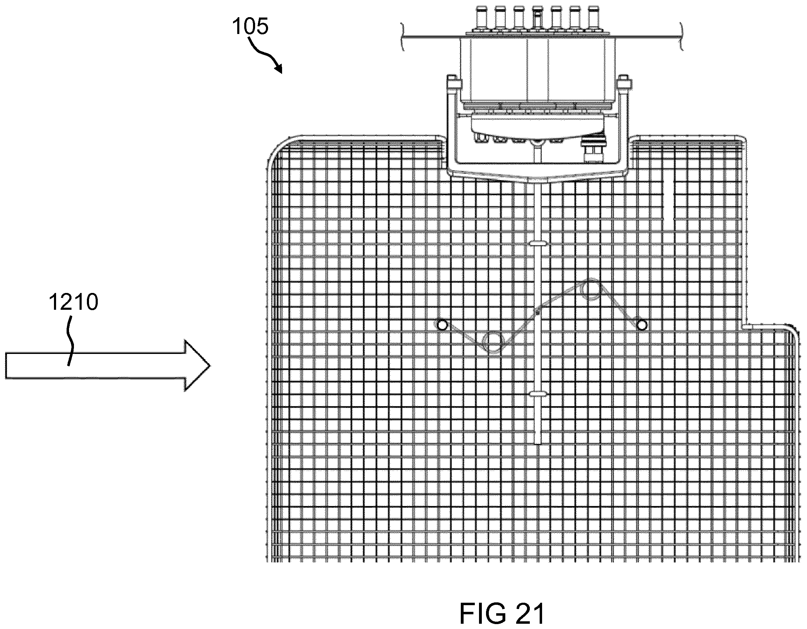

Figur 21 zeigt eine schematische Aufsicht auf eine Kopplungsvorrichtung 105 gemäß einem Ausführungsbeispiel. Dabei handelt es sich um die in der Figur 19 oder 20 beschriebene Kopplungsvorrichtung 105 in dem Kopplungszustand.

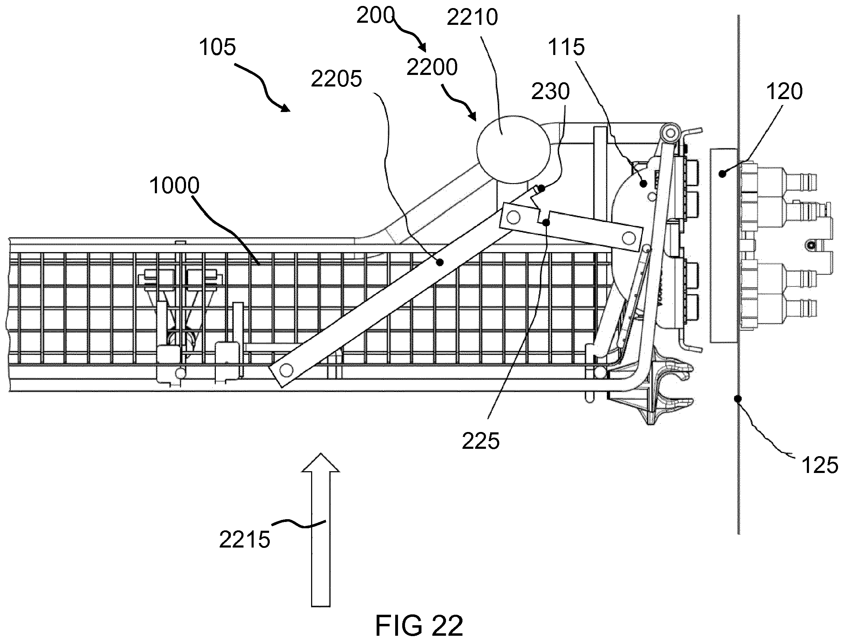

Figur 22 zeigt eine schematische Seitendarstellung einer Kopplungsvorrichtung 105 gemäß einem Ausführungsbeispiel. Dabei kann es sich um die in Figur 1 beschriebene Kopplungsvorrichtung 105 handeln, mit dem Unterschied, dass die Spülgutverbindungseinrichtung 115 und die Geräteverbindungseinrichtung 120 gemäß diesem Ausführungsbeispiel nicht in dem Kopplungszustand miteinander gekoppelt sind. Gemäß diesem Ausführungsbeispiel ist die Spülgutverbindungseinrichtung 115 zudem nicht mit dem Spülgut, sondern mit einem Spülgutträger 1000 in Form eines Spülkorbs verbunden.

Gemäß diesem Ausführungsbeispiel ist die Riegeleinrichtung 200 mit der Spülgutverbindungseinrichtung 115 verbunden. Die Riegeleinrichtung 200 weist gemäß diesem Ausführungsbeispiel eine Hebeleinrichtung auf, die dazu ausgeformt ist, um durch eine manuelle Betätigung die Kopplungsvorrichtung 105 in den Kopplungszustand zu überführen. Die Hebeleinrichtung ist gemäß diesem Ausführungsbeispiel als eine Kniehebeleinrichtung 2200 ausgeformt.

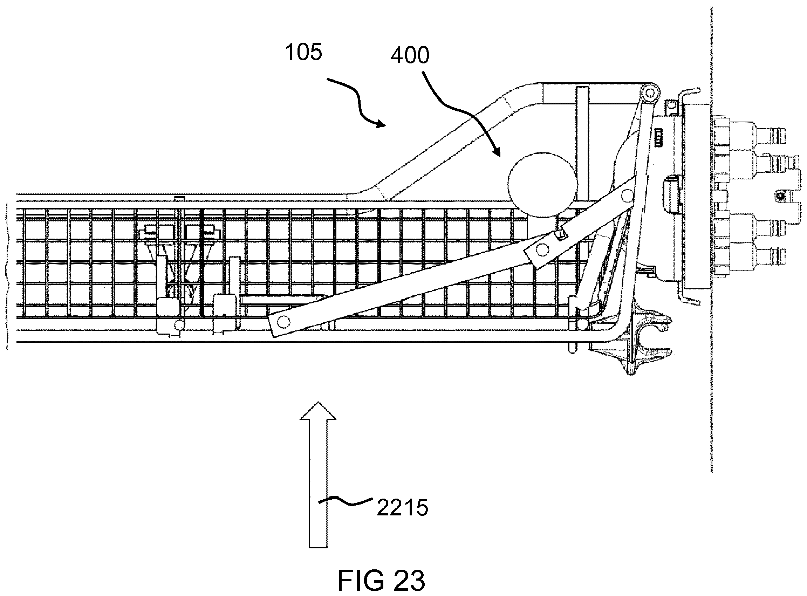

Eine in den Figuren 22 und 23 gezeigte "Lösung 3" der Kopplungsvorrichtung 105 zeigt ein System bestehend aus dem Spülgutträger 1000 und/oder der darin gelagerten oder lagerbaren und geführten oder führbaren Spülgutverbindungseinrichtung 115 in Form eines Anschlussstückes. Die Lagerung des Anschlussstückes ist analog zu der in Figur 12 beschriebenen "Lösung 2" ausgeführt. Anders als derartige bekannte Systeme weist die Spülgutverbindungseinrichtung 115 die Hebeleinrichtung mit der Kniehebeleinrichtung 2200 auf. Die Spülgutverbindungseinrichtung 115 ist gemäß diesem Ausführungsbeispiel mit einem Kniehebelgestänge 2205 der Kniehebeleinrichtung 2200 verbunden, welches wiederum gemäß diesem Ausführungsbeispiel im Spülgutträger 1000 gelagert ist. Das Kniehebelgestänge 2205 weist an einem Ende eine Nase 230 auf, die dazu ausgeformt ist, um mit einer Ausnehmung 225 in Form einer Freimachung zusammenzuwirken. Weiter weist die Kniehebeleinrichtung 2200 gemäß diesem Ausführungsbeispiel einen Kugelgriff 2210 auf, über den der Anwender das Kniehebelgestänge 2205 betätigen und in dessen Endlagen bewegen kann.

Figur 22 zeigt einen eingeschobenen Zustand des Spülgutträgers 1000 in das Reinigungsgerät. In dieser Position befindet sich die Spülgutverbindungseinrichtung 115 genau gegenüber der geräteseitigen und in der Spülraumwand 125 angeordneten Geräteverbindungseinrichtung 120. In dieser Position kann nun der Anwender manuell das Spülgutverbindungseinrichtung 115 über den Kugelgriff 2210 bzw. über das Kniehebelgestänge 2205 in die Geräteverbindungseinrichtung 120 bewegen, wodurch die hydraulische und mechanische Verbindung der Spülgutverbindungseinrichtung 115 zur Geräteverbindungseinrichtung 120 hergestellt ist, siehe hierzu Figur 23.

Ein Pfeil 2215 zeigt eine Einschubrichtung des Spülgutträgers 1000 und/oder der Spülgutverbindungseinrichtung 115.

Figur 23 zeigt eine schematische Seitendarstellung einer Kopplungsvorrichtung 105 gemäß einem Ausführungsbeispiel. Dabei handelt es sich um die in Figur 22 beschriebene Kopplungsvorrichtung 105, mit dem Unterschied, dass die Kopplungsvorrichtung 105 in dem Kopplungszustand 400 angeordnet ist.

Hierzu ist der Kugelgriff manuell nach unten bewegt worden, wodurch die Nase des Kniehebelgestänges in die Ausnehmung des Kniehebelgestänges bewegt wurde, um die Kopplungsvorrichtung 105 zu verriegeln.

In dem Kopplungszustand 400, der auch als Endlage des Kniehebelgestänges bezeichnet werden kann, greift die Nase in die Freimachung und bildet somit einen Anschlag. In dieser Position ist das Kniehebelgestänge selbsthemmend, wodurch eine mechanisch sichere und dauerhafte Verbindung der Spülgutverbindungseinrichtung und der Geräteverbindungseinrichtung hergestellt ist. In diesem Zustand der Spülgutverbindungseinrichtung und des Kniehebelgestänges kann nach einem Schließen einer Gerätetür ein Aufbereitungsprozess gestartet werden. Zur Entnahme des Spülgutträgers erfolgt das Lösen der Spülgutverbindungseinrichtung durch ein Ziehen des Kugelgriffes in entgegengesetzter Richtung, also nach oben, wodurch die Spülgutverbindungseinrichtung wieder in ihre in Figur 22 gezeigte ursprüngliche Position im Spülgutträger bewegt wird.

Im Vergleich zu einer möglichen motorischen Ankupplungsvorrichtung ergeben sich bei der hier vorgestellten Kopplungsvorrichtung 105 als Hauptvorteile geringere Herstellkosten und es fallen geringere Entwicklungskosten an, da kein Aktor, keine Sensorik, kein Aufwand in der Steuerung sowie ein geringerer Aufwand in der Approbation nötig ist, da keine motorische Automatik benötigt wird, Stichwort "Einklemmschutz", "Arbeitssicherheit", "Mechanische Gefährdung".

Weiter ist es möglich, ein bereits bestehendes und bewährtes Serienkonzept, bestehend aus einem Anschlussstück, zu übernehmen, d. h., der Anwender muss sich in Bezug auf die Verwendung des Anschlussstückes nicht umgewöhnen.

Ein weiterer Vorteil ist, dass nur eine einzige Benutzeraktion für den Kupplungsvorgang erforderlich ist, nämlich das Herunterdrücken des Kniehebelgestänges, da die mechanische Verriegelung der Spülgutverbindungseinrichtung über die Selbsthemmung des Kniehebelgestänges erfolgt und der Kupplungsvorgang mit sehr geringem Kraftaufwand durchführbar ist. Zusätzlich erhält der Anwender durch den Anschlag des Kniehebelgestänges, also die Nase, eine haptische Rückmeldung über den sicheren und erfolgreichen Kupplungsvorgang.

Die hier und in den vorangehenden Figuren vorgestellte Kopplungsvorrichtung 105 ist vorrangig für Geräte einsetzbar, die zumindest einen Teil der Endoskopaufbereitung erledigten. Die Kopplungsvorrichtung 105 ist zur Verwendung mit chemischen Desinfektoren und/oder Geräten, welche eine manuelle Vorreinigung unterstützen und/oder Trockenschränke und/oder medizinische Aufbereitungsgeräte für die minimalinvasive Chirurgie ausgebildet.

Umfasst ein Ausführungsbeispiel eine "und/oder"-Verknüpfung zwischen einem ersten Merkmal und einem zweiten Merkmal, so ist dies so zu lesen, dass das Ausführungsbeispiel gemäß einer Ausführungsform sowohl das erste Merkmal als auch das zweite Merkmal und gemäß einer weiteren Ausführungsform entweder nur das erste Merkmal oder nur das zweite Merkmal aufweist.

## Patentansprüche

1. Kopplungsvorrichtung (105) zum mechanischen und hydraulischen Koppeln eines Spülguts (110) mit einem Reinigungsgerät (100), wobei die Kopplungsvorrichtung (105) zumindest die folgenden Merkmale aufweist:
eine Spülgutverbindungseinrichtung (115), die eine Schnittstelle zum Anschließen des Spülguts (110) aufweist und mit einer Geräteverbindungseinrichtung (120) koppelbar ausgeformt ist;
die Geräteverbindungseinrichtung (120), die mit dem Reinigungsgerät (100) verbindbar und mit der Spülgutverbindungseinrichtung (115) koppelbar ausgeformt ist; und
eine Riegeleinrichtung (200), die dazu ausgeformt ist, um in einem Kopplungszustand (400) der Kopplungsvorrichtung (105), in dem die Spülgutverbindungseinrichtung (115) mit der Geräteverbindungseinrichtung (120) gekoppelt ist, die Spülgutverbindungseinrichtung (115) mit der Geräteverbindungseinrichtung (120) zu verriegeln.

2. Kopplungsvorrichtung (105) gemäß Anspruch 1, bei der die Riegeleinrichtung (200) mit der Geräteverbindungseinrichtung (120) verbunden ist.

3. Kopplungsvorrichtung (105) gemäß Anspruch 2, bei der die Geräteverbindungseinrichtung (120) eine Buchse (205) zur Aufnahme zumindest einer Komponente der Spülgutverbindungseinrichtung (115) in dem Kopplungszustand (400) aufweist.

4. Kopplungsvorrichtung (105) gemäß einem der Ansprüche 2 bis 3, bei der die Riegeleinrichtung (200) sich durch eine Öffnung der Geräteverbindungseinrichtung (120) erstreckt, wobei die Öffnung und/oder die Riegeleinrichtung (200) sich quer zu einer Einführrichtung erstreckt, entlang der die Spülgutverbindungseinrichtung (115) bei einem Überführen der Kopplungsvorrichtung (105) in den Kopplungszustand (400) in die Geräteverbindungseinrichtung (120) eingeführt wird.

5. Kopplungsvorrichtung (105) gemäß einem der Ansprüche 2 bis 4, bei der die Riegeleinrichtung (200) federbar lagerbar oder gelagert ist und die Spülgutverbindungseinrichtung (115) eine schräge Seite (210) aufweist, die dazu ausgeformt ist, um bei einem Überführen der Kopplungsvorrichtung (105) in den Kopplungszustand (400), die Riegeleinrichtung (200) automatisch vorzuspannen.

6. Kopplungsvorrichtung (105) gemäß Anspruch 1, bei der die Riegeleinrichtung (200) mit der Spülgutverbindungseinrichtung (115) verbunden ist.

7. Kopplungsvorrichtung (105) gemäß Anspruch 6, bei der die Riegeleinrichtung (200) eine Hebeleinrichtung aufweist, die dazu ausgeformt ist, um durch eine manuelle Betätigung die Kopplungsvorrichtung (105) in den Kopplungszustand (400) zu überführen.

8. Kopplungsvorrichtung (105) gemäß Anspruch 7, bei der die Hebeleinrichtung als eine Kniehebeleinrichtung (2200) oder ein Umlegehebel (1005) ausgeformt ist.

9. Kopplungsvorrichtung (105) gemäß einem der vorangegangenen Ansprüche, die eine Nase (230) aufweist, die dazu ausgeformt ist, um im Kopplungszustand (400) in eine Ausnehmung (225) der Kopplungsvorrichtung (105) zu greifen, um die Kopplungsvorrichtung (105) zu verriegeln.

10. Kopplungsvorrichtung (105) gemäß einem der vorangegangenen Ansprüche, bei der die Spülgutverbindungseinrichtung (115) mit einem Spülgutträger (1000) verbindbar ausgeformt ist.

11. Kopplungsvorrichtung (105) gemäß einem der vorangegangenen Ansprüche, bei der die Spülgutverbindungseinrichtung (115) mehrere, insbesondere zumindest drei Fluidleitungen (215) und die Geräteverbindungseinrichtung (120) mehrere, insbesondere zumindest drei weitere Fluidleitungen (220) aufweist, wobei die Fluidleitungen (115) im Kopplungszustand (400) der Kopplungsvorrichtung (105) mit den weiteren Fluidleitungen (220) fluidisch verbunden sind.

12. Spülgutträger (1000) mit einer Spülgutverbindungseinrichtung (115) einer Kopplungsvorrichtung (105) gemäß einem der vorangegangenen Ansprüche.

13. Reinigungsgerät (100) mit einer Kopplungsvorrichtung (105) gemäß einem der Ansprüche 1 bis 11, bei der die Geräteverbindungseinrichtung (120) an einer Spülraumwand (125) des Reinigungsgeräts (100) angeordnet ist.
